Europäisches Patentamt

European Patent Office

Office européen des brevets

(19)

(11) Veröffentlichungsnummer: **0 264 843 B1**

(12) **EUROPÄISCHE PATENTSCHRIFT**

(45) Veröffentlichungstag der Patentschrift: **27.01.93**

(51) Int. Cl.⁵: **A61K 7/021**

(21) Anmeldenummer: **87115131.2**

(22) Anmeldetag: **16.10.87**

(54) **Kosmetische Zubereitungen.**

(30) Priorität: **23.10.86 DE 3636075**

(43) Veröffentlichungstag der Anmeldung:
**27.04.88 Patentblatt 88/17**

(45) Bekanntmachung des Hinweises auf die
Patenterteilung:
**27.01.93 Patentblatt 93/04**

(84) Benannte Vertragsstaaten:
**DE ES FR GB IT**

(56) Entgegenhaltungen:
EP-A- 0 068 311       EP-A- 0 077 959
EP-A- 0 142 695       DE-A- 1 931 735
DE-A- 3 001 438       US-A- 4 192 691

(73) Patentinhaber: **MERCK PATENT GESELL-
SCHAFT MIT BESCHRÄNKTER HAFTUNG
Frankfurter Strasse 250 Postfach 4119
W-6100 Darmstadt(DE)**

(72) Erfinder: **Franz, Klaus-Dieter, Dr.
Insterburger Str. 12
W-6233 Kelkheim(DE)**
Erfinder: **Griessmann, Andrea, Dr.
Ettesterstr. 19
W-6100 Darmstadt(DE)**
Erfinder: **Lenz, Gisela
Luisenstr. 19
W-6080 Gross-Gerau(DE)**
Erfinder: **Möschl, Gernot
Falltorstr. 20
W-6108 Weiterstadt(DE)**

**Beschreibung**

Die Anmeldung betrifft kosmetische Zubereitungen, die zur Verbesserung des Hautfeelings synthetische, trägerfreie Eisenoxidpigmente, die als plättchenförmige Einkristalle vorliegen, enthalten.

In der Kosmetik besteht ein Bedarf an Effektpigmenten, die den Zubereitungen einen metallischen Farbglanz vermitteln und die physiologisch unbedenkliche, in der Kosmetik zugelassene Farbstoffe enthalten. Es ist bekannt, dafür plättchenförmige Pigmente einzusetzen, wie z. B. die in den deutschen Patenten und Patentanmeldungen 19 59 998, 22 44 298 und 23 13 331 beschriebenen Metalloxid/Glimmer-Pigmente. Dabei handelt es sich um dünne Glimmerplättchen, die mit Eisenoxid und ggf. anderen Metalloxiden beschichtet sind.

Neben dem Farbglanz und den damit erzielten ästhetischen Wirkungen spielt für kosmetische Zubereitungen jedoch auch das Verhalten der Zubereitung auf der Haut, das sogenannte "Feeling", eine entscheidende Rolle. In dieser Hinsicht sind die bekannten Kosmetikpigmente noch verbesserungswürdig.

Es wurde nun gefunden, daß überraschenderweiße beim Einsatz von synthetischen, trägerfreien Eisenoxiden, die als plättchenförmige Einkristalle vorliegen, in kosmetischen Zubereitungen besonders hautfreundliche, angenehme Präparate erhalten werden, wobei durch den metallischen Farbglanz auch sehr vorteilhafte ästhetische Wirkungen erzielt werden können.

Gegenstand der Erfindung sind daher kosmetische Zubereitungen, welche zur Verbesserung des Hautfeelings synthetische, trägerfreie Eisenoxidpigmente, die als plättchenförmige Einkristalle vorliegen, enthalten.

Aus der US-A-4,192,691 sind kosmetische Zubereitungen bekannt, die synthetische Eisenoxidpigmente auf Basis von Glimmersubstraten enthalten. Kosmetische Zubereitungen, die durch hydrothermische Behandlung hergestellte stäbchenförmige Eisenoxidpigmente enthalten, sind aus der DE-A 30 01 438 bekannt.

Wahrend die Kosmetikpigmente aus Basis von Glimmer so hergestellt werden, daß auf die Glimmerplättchen Eisenoxid und ggf. andere Oxide als dünne Schicht aufgefällt werden, sind die entsprechend der vorliegenden Erfindung eingesetzten Eisenoxide nicht auf einem plättchenförmigen Träger aufgebracht, sondern liegen selbst als plättchenförmige Kristalle vor. Sie sind daher wesentlich dünner als die Pigmente auf Glimmerbasis und verleihen den kosmetischen Zubereitungen einen wesentlich höheren Farbglanz bezogen auf das Gewicht des zugesetzten Pigments.

Wiederum im Gegensatz zu den Glimmerpigmenten, bei denen auf der Glimmeroberfläche zahllose kleinste Metalloxidkristalle nebeneinander abgeschieden sind und die daher eine, mikroskopisch gesehen, relativ rauhe Oberfläche besitzen, liegen die in den erfindungsgemäßen Zubereitungen eingesetzten Pigmente als Einkristalle vor, die eine sehr glatte Oberfläche aufweisen. Durch diese Eigenschaften besitzen die Pigmente nicht nur einen hohen Brechungsindex und hohen Glanz, sondern auch ein sehr gutes Hautfeeling.

Die erfindungsgemäß verwendeteten Pigmente sind an sich bekannt und wurden auch schon zur Pigmentierung korrosionsschützender und dekorativer Beschichtungen mit ausgeprägtem metallischem Glanz vorgeschlagen, wobei als Beschichtungsmaterial sowohl Lacke als auch Emails, Glasuren, Kunststoffe oder Kunstharze in Frage kommen. Die Pigmente werden in der Regel nach hydrothermalen Verfahren hergestellt. Verfahren zur Herstellung sind beispielsweise beschrieben im US-Patent 2,989,411, dem europäischen Patent 14 382, den europäischen Patentanmeldungen 11 80 881 und 68 311, der deutschen Patentschrift 30 19 404 sowie weiteren in den genannten Schutzrechten zitierten Publikationen. Neben reinen Eisenoxiden sind darin auch Mischoxide beschrieben, die z. B. Oxide von Elementen der II., IV., V. und/oder VI. Haupt- und/oder Nebengruppe des Periodensystems der Elemente oder z. B. Aluminium enthalten. Ebenfalls bekannt ist auch die Reduktion dieser Pigmente zu Magnetit oder anderen Mischoxiden des zweiwertigen Eisen und ggf. die Reoxidation zu Maghämit ($\gamma$-$Fe_2O_3$). Alle diese synthetischen, plättchenförmigen Eisenoxide sind geeignet für die erfindungsgemäßen Zubereitungen.

Die Pigmente haben in der Regel einen Durchmesser in der Größenordnung von etwa 1 bis etwa 50 $\mu$m und eine Dicke von etwa 0,1 bis etwa 1 $\mu$m. Besonders bevorzugt werden Teilchen mit einer Größe von etwa 5 bis etwa 20 $\mu$m eingesetzt.

Aufgrund des guten Glanzes und des geringen Gewichtes der Plättchen werden nur relativ kleine Mengen der Pigmente benötigt. Die absolute Menge hängt zwar von der Art der Zubereitung und dem gewünschten Farbeffekt ab, in der Regel werden jedoch etwa 6 bis etwa 40 Gew.% der Pigmente eingesetzt.

Als Grundlage für die erfindungsgemäßen Zubereitungen werden die für z. B. Lippenstifte, Fettstifte, Cremes, Puder und andere Kosmetika üblichen Stoffe verwendet. Diese sind dem Fachmann bekannt oder können aus Standardwerken wie z. B. H. Janistyn, Handbuch der Kosmetika und Riechstoffe, Hüthig Verlag Heidelberg, entnommen werden.

Die erfindungsgemäßen Zubereitungen enthalten als färbende Bestandteile in jedem Fall zumindest eines der oben genannten synthetischen, trägerfreien Eisenoxidpigmente. Daneben können jedoch weitere Pigmente zugemischt werden, wobei sowohl organische als auch anorganische Absorptionsfarbpigmente, Silberglanzpigmente wie z. B. Wismutoxichlorid, Fischsilber oder Titandioxid-beschichteter Glimmer oder Interferenzfarbglanzpigmente auf Basis von mit Metalloxiden, insbesondere $TiO_2$, beschichteten Glimmerplättchen eingesetzt werden können.

Insbesondere durch die Kombination mit Interferenzfarbpigmenten oder Metallglanzpigmenten werden sehr ansprechende Farbeffekte erzielt, so daß diese Kombinationen bevorzugt sind. Durch die vorliegende Erfindung stehen daher sehr vorteilhafte, neue kosmetische Zubereitungen mit sehr schönen Farbeffekten und einem sehr angenehmen Hautfeeling zur Verfügung.

Beispiel 1

Crayon-Kosmetikstift

Eine bei 80 °C geschmolzene Fettphase, bestehend aus:
40 Gewichtsteilen Cutina LM® (Lippenstiftmasse der Firma Henkel KGaA, Düsseldorf) und 20 Gewichtsteilen Paraffin werden mit 40 Gewichtsteilen eines plättchenförmigen, rot-braun-glänzenden Pigments, das nach dem Beispiel 2 der US-PS 29 89 411 hergestellt wurde, vermischt, ggf. parfümiert und durch Gießen oder Extrudieren zu Minen für Kosmetikstifte geformt und erkalten lassen.

Beispiel 2

Creme-Mascara bestehend aus den folgenden Bestandteilen:

| 10 | Gewichtsteile Stearinsäure; |
|---|---|
| 3,3 | Gewichtsteile Isopropylmyristat; |
| 4,7 | Gewichtsteile Walrat; |
| 1,0 | Gewichtsteile Sorbitanmonooleat; |
| 1,0 | Gewichtsteile ethoxylierter Sorbitanfettsäureester; |
| 0,2 | Gewichtsteile Propylparaben; |
| 3,3 | Gewichtsteile Triethanolamin; |
| 10,0 | Gewichtsteile Magnesiumaluminiumsilicat; |
| 7,5 | Gewichtsteile Carboset 514® (Acrylharz von Goodrich, Cleveland); |
| 2,5 | Gewichtsteile Propylenglykol; |
| 0,1 | Gewichtsteile Paraben; |
| 46,4 | Gewichtsteile demineralisiertes Wasser; |
| 10 | Gewichtsteile plättchenförmiges $Fe_3O_4$, hergestellt nach Beispiel 7 der EP-PS 14 382. |

Die Bestandteile der Fettphase und die der Wasserphase werden separat auf etwa 75 °C erhitzt und die Wasserphase wird unter Rühren langsam in die Fettphase eingebracht. Nach Abkühlen homogenisiert man die Emulsion und rührt das Schwarzpigment ein. Man erhält eine Mascara, die den Wimpern einen seidigen Schimmer verleiht.

Beispiel 3

Loses Gesichtspuder, bestehend aus den Bestandteilen:

| 59   | Gewichtsteile Talkum; |
|------|------------------------|
| 5    | Gewichtsteile Magnesiumstearat; |
| 10   | Gewichtsteile feinteilige Glimmerplättchen; |
| 15   | Gewichtsteile Timiron Super Sparkle MP-148® (Perlglanzpigment auf Basis von mit Metalloxiden beschichteten Glimmerschuppen, bestehend aus 86 Gew.% Glimmer, 14Gew.% $TiO_2$ der Fa. Atlas ICI-Essen); |
| 6    | Gewichtsteile eines rot-braunen, plättchenförmigen $Fe_2O_3$-Pigments hergestellt nach dem in Journal of Colloid and Interface Science 74 (2) Seite 405 (1980) beschriebenen Verfahren; |
| 5    | Gewichtsteile eines Binders bestehend aus 90 Gewichtsteilen Isopropylstearat, 5 Gewichtsteilen Vaseline und 5 Gewichtsteilen Walrat. |

| Nach Belieben Parfüm und Konservierungsmittel. |
|---|

Die Puderbestandteile werden homogen gemischt, das Perlglanzpigment und das Eisenoxidpigment gut eingerührt und anschließend mit dem geschmolzenen und gut vermengten Binder besprüht. Man erhält einen zarten Puder mit samtigem Schimmer kombiniert mit zusätzlichen Glitsereffekt, der ein angenehmes Hautgefühl vermittelt.

Beispiel 4

Kompaktpuder, bestehend aus den folgenden Bestandteilen:

| 49,5 | Gewichtsteile Talkum; |
|------|------------------------|
| 7,5  | Gewichtsteile Kartoffelstärke; |
| 2,5  | Gewichtsteile Magnesiumstearat; |
| 20   | Gewichtsteile Timiron Super Violet® (Metalloxidglimmerpigment enthaltend 51 Gew.% Glimmer, 49 Gew.% $TiO_2$ der Fa. Atlas ICI-Essen); |
| 10   | Gewichtsteile eines plättchenförmigen Magnetitpigments, hergestellt nach der japanischen Offenlegungsschrift 28 700/ 1976; |
| 10,5 | Gewichtsteile eines Binders bestehend aus 85 Gewichtsteilen Isopropylstearat, 5 Gewichtsteilen Walrat, 5 Gewichtsteilen Vaseline, 4 Gewichtsteilen Parfümöl und 1 Gewichtsteil Propylparaben. |

Die trockenen Puderbestandteile werden homogen gemischt und unter weiterem Mischen mit dem geschmolzenen und gut vermengten Binder besprüht. Danach wird bei einem Preßdruck von 40-60 bar gepreßt. Man erhält einen Lidschattenpuder mit sanftem, metallischem Glanz und weichem Hautfeeling.

Beispiel 5

Lippenstift, mit folgender Zusammensetzung:
69 Gewichtsteile einer Grundmasse folgender Zusammensetzung:

```
63,85     Gewichtsteile Rizinusöl;

12,50     Gewichtsteile Bienenwachs;

 8,00     Gewichtsteile Isopropylmyristat;

 7,50     Gewichtsteile Carnaubawachs;

 5,00     Gewichtsteile Lanolin;

 3,00     Gewichtsteile Paraffin dickflüssig;

 0,10     Gewichtsteile Propylparaben;

 0,05     Gewichtsteile Oxynex 2004®(Antioxidans der
          E. Merck, Darmstadt);


   15     Gewichtsteile einer Farbanteigung von organischen/
          anorganischen Farbstoffen in Rizinusöl der Firma
          Siegle-BASF, Stuttgart;

   10     Gewichtsteile eines violetten, plättchenförmigen
          Eisenoxidpigments, hergestellt nach Beispiel 5 der
          EP 68 311;



    6     Gewichtsteile Rizinusöl;


          nach Belieben Parfüm.
```

Die Bestandteile der Grundmasse werden auf etwa 80 °C erhitzt und gut gemischt. Getrennt davon werden die Bestandteile der Farbanteigung unter Erwärmen auf 60 °C in Rizinusöl angeteigt und zur Homogenisierung 1-2mal über einen Walzenstuhl gegeben. Anschließend werden die Grundmasse, zusätzliches Rizinusöl, die Farbanteigung und das Eisenoxidpigment in einer Gießapparatur gemischt, auf etwa 60 °C temperiert und danach parfümiert. Die homogene Schmelze wird in auf 60 °C vorgewärmte Gießformen gegossen und abkühlen lassen. Die Gießlinge werden den Formen kalt entnommen und nach Erwärmen auf Raumtemperatur noch kurz abgeflammt. Die Stifte erzeugen auf der Haut einen gleichmäßigen, metallischen Farbschimmer.

## Patentansprüche

1. Kosmetische Zubereitungen, welche zur Verbesserung des Hautfeelings synthetische, trägerfreie Eisenoxidpigmente, die als plättchenförmige Einkristalle vorliegen, enthalten.

2. Kosmetische Zubereitungen nach Anspruch 1, dadurch gekennzeichnet, daß die trägerfreien Eisenoxidpigmente in Kombination mit Perlglanzpigmenten auf Basis von mit Metalloxiden beschichteten Glimmerschuppen enthalten sind.

3. Kosmetische Zubereitungen nach Anspruch 1, dadurch gekennzeichnet, daß die trägerfreien Eisenoxidpigmente in einer Menge von 6 bis 40 Gew.% enthalten sind.

## Claims

1. Cosmetic formulations which contain, in order to improve the skin feeling, synthetic, carrier-free iron oxide pigments in the form of platelet-shaped mono-crystals.

2. Cosmetic formulations according to Claim 1, characterised in that they contain the carrier-free iron oxide pigments in combination with nacreous pigments based on mica plates coated with metal oxides.

3. Cosmetic formulations according to Claim 1, characterized in that they contain the carrier-free iron oxide pigments in an amount of 6 to 40% by weight.

**Revendications**

1. Préparations cosmétiques qui contiennent des pigments d'oxyde de fer synthétiques exempts de support, sous forme de monocristaux en paillettes, pour améliorer le confort de la peau.

2. Préparations cosmétiques selon la revendication 1, caractérisées en ce que les pigments d'oxyde de fer exempts de support y sont présents en combinaison avec des paillettes de mica recouvertes d'oxydes métalliques.

3. Préparations cosmétiques selon la revendication 1, caractérisées en ce que les pigments d'oxyde de fer exempts de support y sont présents en une quantité comprise entre 6 et 40 % en poids.